# FASCICULE DE BREVET EUROPEEN

(11) **EP 1 764 078 B1**
(45) Date de publication et mention de la délivrance du brevet: **14.05.2008**
(21) Numéro de dépôt: 06119648.1
(22) Date de dépôt: 28.08.2006
(51) Int. Cl.: A61K 8/11, A61K 8/81, A61K 9/50, A61Q 5/10

(54) **Microcapsules à coeur aqueux et leur utilisation en cosmétique**
Mikrokapsel mit wasserhaltigem Kern sowie ihre Verwendung in kosmetischen Zusammensetzungen
Microcapsules with aqueous core and their use in cosmetic compositions

(30) Priorité: 15.09.2005 FR 0552782
(43) Date de publication de la demande: 21.03.2007
(73) Titulaire: L'ORÉAL, 75008 Paris (FR)
(72) Inventeur: Biatry, Bruno, 94300 Vincennes (FR); Simonnet, Jean-Thierry, 94240 Cachan (FR); Lheureux, Eric, 91230 Montgeron (FR)
(74) Mandataire: Kromer, Christophe

(56) Documents cités:
- EP-A- 1 151 741
- FR-A- 2 698 003
- FR-A- 2 846 555
- PATENT ABSTRACTS OF JAPAN vol. 017, no. 391 (C-1087), 22 juillet 1993 (1993-07-22) & JP 05 070321 A (POLA CHEM IND INC; others: 01), 23 mars 1993 (1993-03-23)
- KONNO T ET AL: "Preparation of nanoparticles composed with bioinspired 2-methacryloyloxyethyl phosphorylcholine polymer" BIOMATERIALS, ELSEVIER SCIENCE PUBLISHERS BV., BARKING, GB, vol. 22, no. 13, juillet 2001 (2001-07), pages 1883-1889, XP004245949 ISSN: 0142-9612

## Description

La présente invention concerne des microcapsules à coeur aqueux contenant au moins un polymère hydratant à groupement phosphorylcholine, des compositions cosmétiques ou dermatologiques les contenant et un procédé pour préparer de telles microcapsules.

La microencapsulation d'actifs cosmétiques ou dermatologiques en vue d'une meilleure conservation et/ou d'une libération prolongée et contrôlée est connue.
Des procédés de microencapsulation et les principes auxquels ils font appel sont décrits en détail par exemple dans "Microencapsulation, Méthods and Industrial Application", édité sous la direction de Benita, M. Dekker, 1996.
Dans ces procédés, la paroi de la microparticule est formée à partir de polymères qui va isoler l'actif du milieu extérieur. Ces microcapsules permettent d'encapsuler des taux plus élevés d'actifs hydrosolubles que d'autres systèmes vésiculaires tels que les liposomes, qui sont des nanoparticules formées de doubles couches phospholipidiques entourant un coeur aqueux.

La demande EP-A-1151741 décrit des microcapsules à coeur aqueux formées d'une enveloppe polymérique et/ou cireuse encapsulant un actif. Ces microcapsules sont obtenues par un procédé de préparation d'émulsification multiple-évaporation de solvant. Ce procédé comprend, en première étape, la préparation d'une émulsion primaire eau-dans-huile obtenue par dispersion d'une composition aqueuse contenant l'actif dans une solution organique du polymère et/ou de la cire formant l'enveloppe de la microcapsule, puis en deuxième étape, la dispersion de l'émulsion primaire dans une phase aqueuse externe. Le solvant organique est ensuite éliminé par évaporation.

L'actif utilisé lors de la préparation des microcapsules a une influence sur la qualité de l'émulsion primaire.

L'actif encapsulé peut également modifier les microcapsules au cours du temps. Ainsi, lorsque l'actif à un faible poids moléculaire, par exemple inférieur à 100 g/mole comme le glycérol, il plastifie le polymère formant la paroi des microcapsules ; ces dernières gonflent au cours du temps et finissent pas éclater. Les microcapsules ne sont donc pas stables et il n'est donc pas possible d'encapsuler du glycérol.

De plus, certains actifs polymères comme l'acide hyaluronique ou l'ADN, ou enore certains polymères épaississants comme la gomme de guar forment des solutions aqueuses de viscosité importante à des teneurs relativement faibles (de l'ordre de 1 % en poids dans l'eau). Or ces solutions aqueuses de forte viscosité nuisent à la préparation de l'émulsion primaire car il est difficile, voire impossible, de disperser la phase aqueuse de l'actif ou de l'additif dans le solvant organique. L'émulsion éventuellement obtenue présente des gouttelettes trop grossières qui ne permettent pas de former des microcapsules. Ces actifs trop visqueux ne peuvent être encapsulés qu'à des teneurs très faibles qui ne permettent pas alors d'obtenir des microcapsules ayant une bonne efficacité de l'actif.

Il n'est donc pas évident pour l'homme du métier de pouvoir obtenir de manière satisfaisante des microcapsules encapsulant un actif dans un milieu aqueux, en particulier avec une teneur élevée en actif.

Le but de la présente invention est donc de disposer de microcapsules à coeur aqueux encapsulant un actif hydratant, en particulier en mettant en oeuvre une quantité d'actif hydratant importante permettant ainsi d'obtenir des propriétés d'hydratation améliorées, notamment avec des compositions contenant ces microcapsules.

Les inventeurs ont découvert de manière surprenante qu'il est possible d'obtenir des microcapsules à coeur aqueux présentant des propriétés d'hydratation améliorées en utilisant comme actif encapsulé un polymère acrylique ayant un groupement de type phosphorylcholine.

Il est connu des documents US-A-5468475, EP-A-767212 et EP-A-1163905 des compositions aqueuses contenant un polymère acrylique ayant un groupement de type phosphorylcholine, notamment des compositions cosmétiques hydratantes pour le soin de la peau. Toutefois, ces documents ne décrivent pas de microcapsules à coeur aqueux contenant ledit polymère acrylique ; ils ne suggèrent pas non plus qu'un tel polymère acrylique peut être encapsulé dans des microcapsules.

Le polymère acrylique ayant un groupement de type phosphorylcholine est facilement soluble ou dispersible dans l'eau et permet l'obtention de solution aqueuse ne présentant pas de viscosité élevée, même à des teneurs élevées dudit polymère acrylique, notamment à des teneurs supérieures à 1 % en poids, en particulier pouvant aller jusqu'à 40% en poids.
Il est ainsi possible d'encapsuler ledit polymère acrylique en des teneurs élevées et d'obtenir ainsi des microcapsules présentant une bonne activité d'hydratation.

De plus, l'encapsulation dudit polymère acrylique permet d'améliorer les propriétés cosmétiques des compositions contenant ces microcapsules en comparaison à une composition comprenant ledit polymère non encapsulé : la composition appliquée sur la peau est plus hydratante et moins collante.

La présente invention a donc pour objet des microcapsules comprenant une enveloppe polymérique et/ou cireuse encapsulant un milieu aqueux contenant un polymère acrylique à groupement de type phosphorylcholine.

L'invention a aussi pour objet un procédé de fabrication de microcapsules à coeur aqueux et à enveloppe polymérique et/ou cireuse décrites précédemment par émulsification multiple-évaporation de solvant.

L'invention a également pour objet une composition notamment cosmétique ou dermatologique contenant, dans un milieu physiologiquement acceptable, les microcapsules à coeur aqueux et à enveloppe polymérique et/ou cireuse décrites précédemment.

L'invention a encore pour objet un procédé non thérapeutique de soin ou de maquillage des matières kératiniques comprenant l'application sur les matières kératiniques d'une composition telle que décrite précédemment.

Les microcapsules selon l'invention comprennent un milieu aqueux encapsulé contenant un polymère acrylique à groupement de type phosphorylcholine, appelé dans la suite de description polymère acrylique PC.

Le polymère acrylique PC est avantageusement un polymère hydrosoluble ou hydrodispersible, et de préférence un polymère hydrosoluble.

Par polymère hydrosoluble, on entend un polymère ayant une solubilité d'au moins 0,1 % en poids dans l'eau à 25 °C.
Par polymère hydrodispersible , on entend un polymère apte à se disperser dans l'eau à 25 °C de manière homogène, sans former de phase inhomogène.

Par polymère acrylique à groupement de type phosphorylcholine, on entend un polymère ayant un squelette acrylique et comprenant des groupements pendants (ou chaînes latérales) contenant un groupement de formule (I) suivante : 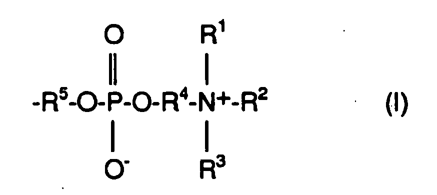 dans laquelle R¹, R² et R³ désignent indépendamment un groupe alkyle ayant de 1 à 8 atomes de carbone ; R⁴ désigne -(CH₂-CHR₆O)ₘ-(CH₂-CHR₆)ₚ- avec R₆ désignant un atome d'hydrogène, un groupe méthyl ou éthyl, et m désigne un nombre entier allant de 0 à 10 et p désigne un nombre entier allant de 1 à 2 ; R⁵ désigne -(CH2)_{g}-, g étant un nombre entier allant de 2 à 10.

Un tel polymère peut être obtenu par polymérisation de monomère acrylique comprenant le groupement de formule (I) décrit précédemment, appelé dans la suite de la description monomère acrylique PC.

Avantageusement, le monomère acrylique PC est un monomère répondant à la formule (II) suivante : 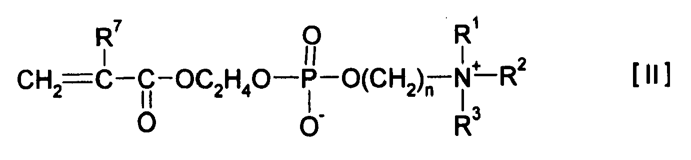 dans laquelle R¹, R² et R³ désignent indépendamment un groupe alkyle ayant de 1 à 8 atomes de carbone ; n représente un nombre entier allant de 2 à 4 ; R⁷ désigne un atome d'hydrogène ou un groupe méthyle.

Comme monomère acrylique PC, on peut citer les monomères suivants :
2-(méth)acryloyloxyéthyl-2'-(triméthylammonio)éthyl phosphate,
3-(méth)acryloyloxypropyl-2'-(triméthylammonio)éthyl phosphate,
4-(méth)acryloyloxybutyl-2'-(triméthylammonio)éthyl phosphate,
5-(méth)acryloyloxypentyl-2'-(triméthylammonio)éthyl phosphaste,
2-(méth)acryloyloxyéthyl-2'-(triéthylammonio)éthyl phosphate,
3-(méth)acryloyloxypropyl-2'(triéthylammonio)éthyl phosphate,
4-(méth)acryloyloxybutyl-2'-(triéthylammonio)éthyl phosphate,
5-(méth)acryloyloxypentyl-2'(triéthylammonio)éthyl phosphate,
2-(méth)acryloyloxyéthyl-2'-(tripropylammonio)éthyl phosphate,
3-(méth)acryloyloxypropyl-2'(tripropylammonio)éthyl phosphate,
4-(méth)acryloyloxybutyl-2'-(tripropylamonnio)éthyl phosphate,
5-(méth)acryloyloxypentyl-2'-(tripropylammonio)éthyl phosphate,
2-(méth)acryloyloxyéthyl-2'-(tributylammonio)éthyl phosphate,
3-(méth)acryloyloxypropyl-2'-(tributylammonio)éthyl phosphate,
4-(méth)acryloyloxybutyl-2'-(tripropylammonio)éthyl phosphate,
5-(méth)acryloyloxypentyl-2'-(tributylammonio)éthyl phosphate,
2-(méth)acryloyloxyéthyl-3'-(triméthylammonio)propyl phosphate,
2-(méth)acryloyloxyéthyl-3'-(triéthylammonio)propyl phosphate,
2-(méth)acryloyloxyéthyl-4'(triéthylammonio)butyl phosphate,
2-(méth)acryloyloxyéthyl-3'-(tripropylammonio)propyl phosphate,
2(méth)acryloyloxyéthyl-4'-(tripropylammonio)butyl phosphate,
2-(méth)acryloyloxyéthyl-3'-(tributylammonio)propyl phosphate,
2-(méth)acryloyloxyéthyl-4'-(tributylammonio)butyl phosphate,
3-(méth)acryloyloxyéthyl-3'(triméthylammonio)propyl phosphate,
3-(méth)acryloyloxypropyl-4'-(triméthylammonio)butyl phosphate,
3-(méth)acryloyloxypropyl-3'(triéthylammonio)propyl phosphate,
3-(méth)acryloyloxypropyl-4'-(triéthylammonio)butyl phosphate,
3-(méth)acryloyloxypropyl-3'-(tripropyllammonio)propyl phosphate,
3-(méth)acryloyloxypropyl-4'-(tripropylammonio)butyl phosphate,
3-(méth)acryloyloxypropyl-3'-(tributylammonio)propyl phosphate,
3-(méth)acryloyloxypropyl-4'-(tributylammonio)butyl phosphate,
4-(méth)acryloyloxybutyl-3'-(triméthtylammonio)propyl phosphate,
4-(méth)acryloyloxybutyl-4'-(triméthylammonio)butyl phosphate,
4-(méth)acryloyloxybutyl-4'-(triehtylammonio)butyl phosphate,
4-(méth)acryloyloxybutyl-3'-(tripropylammonio)propyl phosphate,
4-(méth)acryloyloxybutyl-4'-(tripropylammonio)butyl phosphate,
4-(méth)acryloyloxybutyl-3'-(tributylammonio)propyl phosphate, et
4-(méth)acylolyloxybutyl-4'(tribuylammonio)butyl phosphate.

Comme monomère acrylique PC, on utilise de préférence le 2-(méth)acryloyloxyéthyl-2'-(triméthylammonio)éthyl phosphate, appelé également 2-(méthacryloyloxyéthyl)phosphorylcholine.

De préférence, le polymère acrylique PC utilisé selon l'invention est un polymère obtenu par polymérisation d'un monomère acrylique PC tel que décrit précédemment et éventuellement d'un ou plusieurs monomères additionnels différent du monomère acrylique PC.

Les monomères additionnels peuvent être choisis parmi le (méth)acrylate de méthyle, le (méth)acrylate d'éthyle, le (méth)acrylate de butyle, l'acide (méth)acrylique, le (méth)acrylamide, le (méth)acrylate de 2-hydroxyéthyle, l'éthyl vinyl éther, le butyl vinyl éther, la N-vinylpyrrolidone, le chlorure de vinyle, l'éthylène, l'isobutylène, l'acrylonitrile, le styrène, le méthyl styrène, le chlorométhyl styrène.

Le polymère acrylique PC peut comprendre de 40 à 100 % en mole d'unités issues du monomère acrylique PC tel que décrit précédemment et de 0 à 60 % en mole d'unités issues de monomère additionnel.

De manière préférée, le polymère à groupement de type phosphorylcholine est choisi parmi l'homopolymère de 2-(méthacryloyloxyéthyl)phosphorylcholine, le copolymère 2-(méthacryloyloxyéthyl)phosphorylcholine/méthacrylate de butyle, le copolymère 2-(méthacryloyloxyéthyl)phosphorylcholine/chlorure de 2-hydroxy-3-méthacryloyloxypropyltriméthylammonium, le terpolymère de 2-(méthacryloyloxyéthyl) phosphorylcholine/méthacrylate de butyle/méthacrylate de sodium , le copolymère 2-(méthacryloyloxyéthyl) phosphorylcholine/méthacrylate de stéaryle.
De préférence, on utilise l'homopolymère de 2-(méthacryloyloxyéthyl) phosphorylcholine ou le copolymère 2-(méthacryloyloxyéthyl) phosphorylcholine/méthacrylate de butyle, et plus préférentiellement l'homopolymère de 2-(méthacryloyloxyéthyl)phosphorylcholine.

De tels polymères sont décrits dans les documents EP-A-1163905, EP-A-1095665, FR-A-2698003, EP-A-767212, dont le contenu est incorporé à titre de référence dans la présente demande.

Le polymère acrylique PC a de préférence un poids moléculaire en poids allant de 50 000 à 1 000 000, et préférentiellement allant de 80 000 à 800 000.

Comme polymère acrylique à groupement phosphorylcholine conforme à l'invention, on peut utiliser :
- le poly-2-(méthacryloyloxyéthyl)phosphorylcholine à 40 % dans un mélange eau/butanediol (5% de butanediol) vendu sous la dénomination LIPIDURE HM par la société Nippon Oils and Fats ; ce produit a pour nom CTFA : polyphosphorylcholine glycol acrylate (and) butylene glycol.
- le copolymère de 2-(méthacryloyloxyéthyl) phosphorylcholine/méthacrylate de butyle (90/10) à 5 % en solution dans l'eau vendu sous la dénomination LIPIDURE PMB par la société Nippon Oils and Fats ; ce produit a pour nom CTFA : POLYQUATERNIUM-51.
- le copolymère de 2-(méthacryloyloxyéthyl)phosphorylcholine/chlorure de 2-hydroxy-3-méthacryloyloxypropyltriméthylammonium à 5% en solution dans l'eau, vendu sous la dénomination LIPIDURE-C par la société Nippon Oils and Fats.
- le terpolymère de 2-(méthacryloyloxyéthyl) phosphorylcholine/méthacrylate de butyle/méthacrylate de sodium à 5 % en solution dans l'eau, vendu sous la dénomination LIPIDURE-A par la société Nippon Oils and Fats.
- les copolymères de 2-(méthacryloyloxyéthyl) phosphorylcholine/méthacrylate de stéaryle vendus sous les dénominations LIPIDURE-S, LIPIDURE-NR, LIPIDURE-NA par la société Nippon Oils and Fats ; ces produits ont pour nom CTFA : polyquaternium-61.

Le polymère acrylique PC peut être présent dans le milieu aqueux encapsulé dans les microcapsules en une teneur allant de 0,1 % à 40 % en poids, par rapport au poids total de la phase aqueuse encapsulée, de préférence allant de 1 % à 40 % en poids, de préférence encore allant de 2 % à 40 % en poids, de préférence allant de 5 % à 40 % en poids, préférentiellement allant de 5 % à 30 % en poids, et plus préférentiellement allant de 10 % à 25 % en poids.

L'enveloppe des microcapsules (ou paroi) peut comprendre au moins un polymère insoluble dans l'eau et/ou une cire.

Par polymère insoluble dans l'eau on entend un polymère dont la solubilité dans l'eau à 25 °C est inférieure à 0,1 % en poids.

Le polymère insoluble dans l'eau de l'enveloppe peut être choisi parmi :
- la polycaprolactone (comme celle vendue sous la dénomination CAPA640 par la société SOLVAY), la polybutyrolactone, le poly(3-hydroxybutyrate) ;
- les poly (alkylène C2-C6 adipate) comme le poly(éthylène adipate) ou le poly(butylène adipate).

Le terme de poly(alkylène adipate) englobe à la fois les homopolymères d'acide adipique et d'un alcane-diol et les copolymères de type poly(ester éther), linéaires ou ramifiés, obtenus à partir d'acide adipique et d'un ou de plusieurs alcane-diols et/ou éthers-diols et/ou triols.

Les alcane-diols utilisés pour la préparation desdits poly(alkylène adipate) sont des alcane-diols en C2-6 à chaîne linéaire ou ramifiée choisis parmi l'éthylèneglycol, le propylèneglycol, le 1,3-propanediol, le 1,4-butanediol, le 1,5-pentanediol, le 1,6-hexanediol et le néopentylglycol.
Les éther-diols sont des di-, tri- ou tétra-(alkylène en C2-4)-glycols tels que le diéthylèneglycol, le triéthylèneglycol, le tétraéthylèneglycol, le dipropylèneglycol, le tripropylèneglycol, le tétrapropylèneglycol ou le dibutylèneglycol, le tributylèneglycol ou le tétrabutylèneglycol.

Comme indiqué ci-dessus, les polyesters de type poly(alkylène adipate) utilisés pour la préparation des microcapsules de l'invention peuvent également contenir un nombre limité de motifs de ramification dérivés de triols.

Les triols utilisés sont généralement choisis parmi le glycérol, le triméthyloléthane et le triméthylolpropane.

Des polymères de poly(alkylène adipate) sont notamment décrits dans la demande EP-A-1029587.
- les polyesters polyols d'acide adipique et de butanediol comme le polyester d'acide adipique, de 1,4-butanediol et de 2-éthyl-2-(hydroxyméthyl)-1,3-propanediol (comme le LEXOREZ® 1151-35 par la société INOLEX). Des polyesters polyols d'acide adipique et de butanediol sont notamment décrits dans la demande EP-A-1342471.
- les esters de cellulose et d'au moins un acide carboxylique en C₁-C₄, comme l'acétate de cellulose, l'acétopropionate de cellulose (par exemple ceux vendus sous les dénominations "CAP-482-0,5", "CAP-482-20" et "CAP-504" par la société Eastman Chemical), l'acétobutyrate de cellulose (par exemple ceux vendus sous les dénominations "CAB-551 ", "CAB-500", "CAB 553", "CAB-381 "par la société Eastman Chemical) , et de préférence parmi l'acétobutyrate de cellulose et l'acétopropionate de cellulose ;
- les poly(ortho ester) obtenus par polycendensation de polyol, de diol lactide et de 3,9-diéthylidène-2,4,8,10-tétraoxaspiro-[5.5]-undécane , tels que ceux décrits dans la publication Bouchemal K., Microencapsulation of dehydroepiandrosterone (DHEA) with poly(ortho ester) polymers by interfacial polycondensation, Journal of microencapsulation, 2003, vol 20, n°5, 637-651 ;
- les polymères blocs poly(éthylèneglycol)-poly(butylene téréphatale) tels que ceux décrits dans la publication Bezemer J.M., Microsphères for protein delivery prepared from apmhiphilic multiblok copolymers, Journal of Controlled Release, 67 (2000) 233-248 ;
- les copolymères polyéthylène glycol téréphtalate/polybutylène téréphatale, tels que ceux décrits dans le brevet US 5980948 ;
- les copolymères de styrène et d'anhydride maléique, tels que ceux vendus sous la dénomination SMA par la société Cray-Valley ;
- les copolymères de styrène et d'acide acrylique, tels que celui vendu sous la dénomination Plioway Ultra 200 par al socitété Plioway ;
- les terpolymères séquencés styrène-éthylène/butylène-styrène, les terpolymères séquencés styrène-éthylène/propylène-styrène, tels que ceux vendus sous la dénomination KRATON G par la société Shell ;
- les terpolymères d'éthylène, d'acétate de vinyle et d'anhydride maléique, tels que ceux vendu sous la dénomination AREVAC par la société Arkema.
- et leurs mélanges.

La cire de l'enveloppe peut être choisie parmi la cire d'abeille, la cire d'abeille polyglycérolée, les huiles végétales hydrogénées, la paraffine de point de fusion supérieur à 45 °C, et les cires de silicone.

A titre de cires de silicone, on peut citer par exemple les alkyl- ou alcoxydiméthicones comprenant de 16 à 45 atomes de carbone, comme la béhénoxydiméthicone et les alkylesters de diméthiconol en C16-45 comme le diméthiconol béhénate.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope.

Les microcapsules selon la présente invention permettent d'obtenir des taux d'encapsulation du polymère acrylique PC supérieur ou égal à 25 % voire supérieurs à 60 %, par rapport au poids de l'actif mis en oeuvre.

Le milieu aqueux encapsulé dans les microcapsules peut comprendre un polymère hydrosoluble additionnel différent du polymère à groupement PC décrit précédemment.

Les polymères hydrosolubles additionnels peuvent être choisis notamment parmi l'alcool polyvinylique), la polyvinylpyrrolidone, la carboxyméthylcellulose, les poly(acide carboxylique) et les dérivés réticulés de ceux-ci, et les gommes naturelles telles que les xanthanes, l'amidon, l'alginate de sodium, les pectines, le chitosane, le guar, le caroube, le carraghénane, l'acide hyaluronique. Ces polymères hydrosolubles additionnels peuvent être présents en une teneur allant de 0,01 à 10 %, de préférence à raison de 0,1 à 5 % en poids, par rapport au poids total du milieu aqueux encapsulé.

Le milieu aqueux encapsulé peut comprendre un sel pouvant être choisi parmi le chlorure de sodium, le chlorure de potassium, le sulfate de magnésium et le chlorure de magnésium. Le sel peut être présent en une teneur allant de 0,1 à 10 % en poids, par rapport au poids total du milieu aqueux encapsulé, et de préférence allant de 0,1 et 5 % en poids.

Les microcapsules peuvent en particulier être univacuolaires ou multivacuolaires, c'est-à-dire l'enveloppe externe peut renfermer un seul compartiment de phase aqueuse ou bien la phase aqueuse interne peut être divisée en une multitude de compartiments séparés par des parois de même nature chimique que l'enveloppe externe. Ce phénomène se produit généralement lorsque l'émulsion multiple est particulièrement stable et donne d'excellents résultats d'encapsulation.

Le rapport en poids du milieu aqueux encapsulé formant le coeur des microcapsules de la présente invention à l'enveloppe de celles-ci (polymère et/ou cire) est généralement compris entre 0,1/1 et 50/1 et de préférence entre 0,5/1 et 10/1.

Les microcapsules de la présente invention ont généralement un diamètre moyen compris entre 1 µm et 1000 µm et plus particulièrement entre 1 et 80 µm.

La présente invention a également pour objet un procédé de fabrication des microcapsules à coeur aqueux contenant un polymère acrylique PC et une enveloppe polymérique et/ou cireuse telles que décrites précédemment. Ce procédé est un procédé de microencapsulation par émulsion multiple-évaporation de solvant comportant les étapes successives suivantes :
(a) solubilisation d'au moins un polymère acrylique PC dans un milieu aqueux,
(b) émulsification de la solution aqueuse obtenue dans l'étape (a) dans une solution d'au moins un polymère insoluble dans l'eau et/ou d'au moins une cire dans un solvant organique non miscible à l'eau,
(c) émulsification de l'émulsion primaire eau-dans-huile obtenue dans l'étape (b) dans une solution aqueuse contenant de préférence un agent stabilisant de l'émulsion,
(d) élimination du solvant organique par évaporation donnant une suspension aqueuse de microcapsules.

La solution aqueuse préparée à l'étape (a) contient avantageusement le polymère acrylique PC en une teneur similaire à celles décrites précédemment pour le milieu aqueux encapsulé dans les microcapsules.

La nature du solvant organique non miscible à l'eau utilisé dans l'étape (b) est généralement choisie en fonction de son pouvoir solvant vis-à-vis du matériau de la paroi, de sa solubilité dans l'eau qui doit être la plus faible possible et de son point d'ébullition qui est de préférence inférieur à 100 °C. On peut utiliser par exemple le dichlorométhane, le cyclohexane, l'heptane, le chloro-1-butane, l'acétate d'éthyle, le formate d'éthyle, le diméthoxyméthane (ou méthylal).

La stabilité de l'émulsion multiple est un facteur déterminant pour l'obtention de bons résultats d'encapsulation. En effet, une stabilité insuffisante de l'émulsion multiple entraînerait un mélange des phases aqueuses interne et externe et une fuite de l'actif hors des vésicules formées. Il est par conséquent fortement recommandé d'ajouter à la phase aqueuse continue de l'étape (c) un agent stabilisant de l'émulsion.

Des agents stabilisants polymériques appropriés sont connus dans la technique et peuvent être choisis par exemple parmi le poly(alcool vinylique), la polyvinylpyrrolidone, les copolymères hydrosolubles styrène-anhydride maléique, la carboxyméthylcellulose, l'amidon, le chitosane et l'acide polyacrylique.

Bien que l'utilisation d'un agent stabilisant polymérique soit préférable, on peut également utiliser à la place de celui-ci des agents tensioactifs hydrosolubles.

En vue d'augmenter la stabilité de l'émulsion multiple, la phase aqueuse continue de celle-ci peut également contenir de 0,1 à 10 % en poids, par rapport au poids total de ladite phase aqueuse continue, d'un sel minéral choisi par exemple parmi le chlorure de sodium, le chlorure de potassium, le sulfate de magnésium et le chlorure de magnésium.

On pourra également introduire dans la solution de solvant organique de l'étape (b) un agent tensio-actif afin d'améliorer la stabilité de l'émulsion primaire. L'homme du métier saura choisir le composé adéquat, comme par exemple des agents tensio-actifs de HLB (balance hydrophile-lipophile) inférieure à 10, tels que les esters de sorbitane et d'acides gras comme par exemple les polysorbates, les lécithines liposolubles, les monoglycérides d'acides gras, le PEG-30 dipolyhydrostéarate (ARLACEL® P135 de la société ICI), le cétyldiméthicone-copolyol (ABIL® EM90 de la société GOLDSCHMIDT), le polydiméthylsiloxane oxyéthyléné (DC2-5695® de la société DOW CHEMICAL) , les polyoléfines à terminaison succinique (Lubrizol 5603® de la société LUBRIZOL, Chemcinnate 1000 AF ou 2000 de la sociétéCHEMRON), les glycoglycérides tels que ceux décrits dans EP-A-1151745.

Le procédé d'émulsification multiple-évaporation du solvant est déjà connu des brevets US 3523906 et US 3523907 et de la demande EP-A-1151741.

La taille et la structure interne des microcapsules dépend d'un très grand nombre de paramètres liés au procédé de fabrication, tels que la température, la vitesse d'agitation lors de l'émulsification, la nature chimique et les quantités respectives des différents composants hydrosolubles et organosolubles, la quantité d'agents stabilisants etc. L'homme du métier saura faire varier ces différents paramètres pour obtenir la morphologie de microcapsules recherchée.

La suspension de microcapsules obtenue après évaporation du solvant peut être utilisée telle quelle ou être incorporée dans une composition cosmétique ou dermatologique.

Si on le souhaite, les microcapsules peuvent également être séparées à partir de la suspension aqueuse obtenue dans l'étape (d) par filtration et on peut les sécher de manière à obtenir une poudre de microcapsules.

L'invention concerne également une composition comprenant dans un milieu physiologiquement acceptable, les microcapsules décrites précédemment.

On entend par milieu physiologiquement acceptable un milieu compatibles avec les matières kératiniques d'êtres humains. Il s'agit de préférence d'un milieu cosmétiquement ou dermatologiquement acceptable , c'est-à-dire présente une couleur, une odeur et un toucher agréables et qui ne génère pas d'inconforts inacceptables (picotements, tiraillements, rougeurs) susceptibles de détourner la consommatrice d'utiliser cette composition.

Les microcapsules (coeur et paroi) peuvent être présentes dans la composition selon l'invention en une teneur allant de 0,1 % à 30 % en poids, par rapport au poids total de la composition, et de préférence allant de 0,1 % à 10 % en poids.

Le polymère acrylique PC peut être présent dans la composition selon l'invention en une teneur allant de 0,01 % à 10 % en poids, par rapport au poids total de la composition, et de préférence allant de 0,01 % à 5 % en poids.

La composition selon l'invention peut comprendre de l'eau et/ou un solvant organique miscible à l'eau à 25 °C et/ou une huile.

La composition selon l'invention peut comprendre un solvant organique miscible à l'eau à 25 °C notamment choisi parmi les alcools inférieurs en C₁-C₆ tels que l'éthanol, l'isopropanol, le propanol, le butanol ; les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

La composition selon l'invention peut comprendre une huile, notamment choisie parmi :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.

Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10. On peut également utiliser les esters d'acides di- ou tri-carboxyliques en C₄-C₂₂ et d'alcools en C₁-C₂₂ et les esters d'acides mono-, di- ou tri-carboxyliques et d'alcools di-, tri-, tétra- ou penta-hydroxylés en C₂-C₂₆.

Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanate d'isononyle ; l'octanoate de cétyle.

La composition selon l'invention peut se présenter sous toutes les formes galéniques classiquement utilisées pour une application topique et notamment sous forme d'émulsion (notamment d'émulsion huile dans eau ou eau dans huile, ou d'émulsion multiple(E/H/E ou H/E/H)), de gel aqueux ou huileux, de lotion aqueuse ou huileuse, de poudre, de stick, de spray, de mousse. Ces compositions sont préparées selon les méthodes usuelles.

Selon un mode préféré de réalisation de l'invention, la composition se présente sous la forme d'une émulsion et plus particulièrement d'une émulsion huile-dans-eau (H/E).

La composition peut en outre contenir divers adjuvants couramment utilisés dans le domaine cosmétique, notamment choisis parmi les émulsionnants, les cires, les charges, les pigments, les matières colorantes, les séquestrants, les parfums, les épaississants, les conservateurs, les actifs cosmétiques ou dermatologiques, les filtres UV, les agents hydratants, les polymères filmogènes.

Bien entendu, l'homme du métier veillera à choisir ces adjuvants et/ou leur quantité de manière telle que les propriétés avantageuses de la composition selon l'invention ne soient pas, ou substantiellement pas, altérées par l'adjonction envisagée.

La composition selon l'invention peut être une composition de soin ou de maquillage du visage et/ou du corps. Elle est de préférence sous la forme d'une composition non rincée. Il peut ainsi s'agit d'une composition de soin du visage et/ou du corps, ou d'une composition de maquillage telle qu'un produit du teint (notamment fond de teint), un fard à paupières, un fard à joue, un eye-liner, un produit anticernes ou un produit de maquillage du corps.

La composition peut également être une composition capillaire, tel qu'un shampooing, un après shampoing, une composition de coiffage, une coloration capillaire.

L'invention va maintenant être décrite en référence aux exemples suivants donnés à titre illustratif et non limitatif.

### Exemple 1 :

On a préparé des microcapsules de polymère acrylique PC selon le mode opératoire suivant :

On a dilué avec de l'eau une solution aqueuse de poly-2-(méthacryloyloxyéthyl)phosphorylcholine à 40 % dans un mélange eau/butanediol (5% de butanediol) vendu sous la dénomination LIPIDURE HM par la société Nippon Oils and Fats jusqu'à une teneur de 20 % en poids de polymère. Dans un réacteur on a émulsionné 161,8 ml de cette solution aqueuse dans 200 ml de dichlorométhane contenant 29,4 g d'acétopropionate de cellulose (CAP-482-0,5® de la société Eastman Chemical) à l'aide d'un homogénéiseur de type rotor-stator pendant 5 minutes à 24000 tour par minute en maintenant la température du mélange en dessous de 25 °C.
160,6 ml de l'émulsion primaire ainsi obtenue a été introduite dans 500 ml d'une solution aqueuse à 1% en poids d'alcool polyvinylique (Celvol 203®, Celanese) et à 7 % de chlorure de sodium, maintenue sous agitation à l'aide d'un disperseur Moritz pendant 20 minutes à 2000 tour par minute, à une température de 25°C.

Le dichlorométhane est ensuite évaporé sous vide (Rotavapor Büchi B-480) à 40°C. Après décantation et filtration on a récupère les microparticules formées. Les microcapsules ont un diamètre moyen égal à 54 µm.

On a mesuré le taux d'encapsulation par dosage du polymère acrylique PC après minéralisation et dosage du phosphore à l'aide du kit Biomerieux (Lyon, France) « Phosphore UV».
Le taux d'encapsulation correspond au rapport de la quantité d'actif retrouvée à l'intérieur des microcapsules sur la quantité totale d'actif mise en oeuvre lors de la préparation des microcapsules.

Les microcapsules obtenues ont un taux d'encapsulation du poly-2-(méthacryloyloxyéthyl)phosphorylcholine égal à 92 % (= 17,8/19,6).

### Exemples 2 et 3 comparatifs :

On a préparé une composition de soin de la peau (exemple 2) comprenant les ingrédients suivants :

| | |
|---|---|
| Microcapsules selon l'exemple 1 | 5,6 g* |
| Acide polyacrylamidométhyl propane sulfonique neutralisé partiellement à l'ammoniaque et réticulé | |
| (HOSTACERIN AMPS de la société CLARIANT) | 0,5 g |
| Conservateur | qs |
| Eau qsp | 100 g |

| | |
|---|---|
| * soit 1 % en matière active de polymère à groupe PC | |

On a préparé une composition similaire à la précédente mais dans laquelle le polymère acrylique PC (Lipidure HM) n'est pas encapsulé mais se trouve directement dans la phase aqueuse continue. La composition (exemple 3) est la suivante :
- poly-2-(méthacryloyloxyéthyl)phosphorylcholine à 40 %
   dans un mélange eau/butanediol (5% de butanediol) vendu sous la dénomination LIPIDURE HM par la société Nippon Oils and Fats 2,5 g soit 1 % MA
- Eau 96,9 g
- Acide polyacrylamidométhyl propane sulfonique neutralisé partiellement à l'ammoniaque et réticulé (HOSTACERIN AMPS de la société CLARIANT) 0,5 g
- Conservateur qs

On a évalué les propriétés hydratantes des 2 compositions sur un panel de 25 femmes sélectionnées pour avoir une peau au niveau des jambes ayant une Perte Insensible en Eau (PIE) élevée.

Le test est effectué en comparant l'état d'hydratation d'une zone de peau traité par rapport à une zone de peau non traitée (dite peau nue).

Les 2 formules ont été appliquées deux fois par jour pendant 2 semaines sur les jambes.
L'hydratation a été mesurée par cornéométrie (mesure de l'impédance électrique (IE) de la peau avec un Cornéomètre CM825 SEI-M-0152-COMB- 03) à T0 et T15 jours.
On a déterminé le rapport (IE peau traitée - IE peau nue)/IE peau nue à T15 jours obtenu pour chaque composition ; on a obtenu les résultats suivants, exprimé en pourcentage :

| | (IE peau traité - IE peau nue)/ IE peau nue |
|---|---|
| Exemple 2 (invention) | + 9,2 % |
| Exemple 3 (hors invention) | + 3,9 % |

Les résultats obtenus montrent que la composition de l'exemple 2 selon l'invention a une propriété hydratante supérieure à celle de la composition de l'exemple 3. Ce résultat confirme que l'encapsulation du polymère acrylique PC permet d'améliorer l'activité hydratante du polymère.

Par ailleurs, le panel de femmes a relevé que la composition de l'exemple 3 ne faisant pas partie de l'invention est peu hydratante, collante et inconfortable. L'encapsulation du polymère acrylique PC permet donc d'améliorer les propriétés de la composition contenant un tel polymère.

### Exemple 4 :

On a préparé des microcapsules de polymère acrylique PC et de hyaluronate de sodium selon le mode opératoire suivant :

On a dilué avec de l'eau une solution aqueuse de poly-2-(méthacryloyloxyéthyl)phosphorylcholine à 40 % dans un mélange eau/butanediol (5% de butanediol) vendu sous la dénomination LIPIDURE HM par la société Nippon Oils and Fats jusqu'à une teneur de 10 % en poids de polymère puis on a ajouté 0,5 % en poids de hyaluronate de sodium vendu sous la dénomination Cristalhyal® par la société Soliance. Dans un réacteur, on a émulsionné 160 ml de solution aqueuse ainsi préparée dans 200 ml de dichlorométhane contenant 20 g de d'acétopropionate de cellulose (CAP-482-0.5® de la société Eastman Chemical) à l'aide d'un homogénéiseur de type rotor-stator pendant 5 minutes à 24000 tour par minute en maintenant la température du mélange en dessous de 25°C. 250ml de l'émulsion primaire ainsi obtenue a été introduite dans 500 ml d'une solution aqueuse comprenant 1% en poids d'alcool polyvinylique (Celvol 203®, Celanese) et 0,5 % en poids de chlorure de sodium, maintenue sous agitation à l'aide d'un disperseur Moritz pendant 20 minutes à 2000 tour par minute, à une température de 25°C.
Le dichlorométhane a été ensuite évaporé sous vide et on a récupéré les microcapsules formées après décantation et filtration. Les microcapsules ont un diamètre moyen égal à 49 µm. Le taux d'encapsulation du polymère acrylique à groupe PC est de 70%.

La composition de la poudre de microcapsules obtenue est la suivante :

| | |
|---|---|
| Acéto-propionate de Cellulose (CAP-482-0.5® de la Sté Eastman Chemical) | 11,1 g |
| poly-2-(méthacryloyloxyéthyl)phosphorylcholine (Lipidure HM® de la Sté NOF) | 22,2 g MA |
| Hyaluronate de sodium (Cristalhyal® de la Sté Soliance) | 0,33 g |
| Eau déminéralisée | 66,37 g |

### Exemple 5 :

On a préparé une composition de soin de la peau sous forme d'émulsion H/E contenant les ingrédients suivants :

| Phase A | |
|---|---|
| PEG-20 méthyl glucose sesquistéarate (GLUCAMATE SSE 20 de chez Noveon) | 2 g |
| Sel disodique de l'EDTA | 0,1 g |
| Glycérine | 3 g |
| Conservateur | 0,2 g |
| Eau déminéralisée | 53,5 g |
| | |

| Phase B | |
|---|---|
| Méthyl glucose sesquistéarate (Glucate SS de chez Noveon) | 2 g |
| Mélange d'alcool stéarylique et de Ceteareth-20 (RITAPRO 200 de la société Rita) | 2 g |
| Cyclohexasiloxane | 6 g |
| Stéarate d'isocétyle | 8 g |
| Dicaprylyl carbonate | 3 g |
| Filtres UV | 4,9 g |
| Conservateur | 0,1 g |
| | |

| Phase C | |
|---|---|
| Copolymère acrylamide/acrylamido 2-méthyl propane sulfonate de sodium en émulsion inverse à 40 % dans isoparrafine/eau (Sepigel 305 de Seppic) | 1 g |
| Acide polyacrylamidométhyl propane sulfonique neutralisé partiellement à l'ammoniaque et réticulé (Hostacerin AMPS de Clariant) | 1,2 g |
| | |

| Phase D | |
|---|---|
| Aluminum Starch Octenylsuccinate | 3 g |
| | |

| Phase E | |
|---|---|
| Microparticules de l'exemple 4 | 10 g |

L'émulsion est préparée selon les techniques connues d'émulsification.
La composition appliquée sur la peau confère de bonnes propriétés d'hydratation et de douceur.

### Exemple 6 : émulsion H/E contenant les microcapsules de l'exemple 4

| Phase A | |
|---|---|
| Eau déminéralisée | 40,69 g |
| Sel disodique de l'acide éthylène diamine tétracétique | 0,1 g |
| Glycerin | 3 g |
| Conservateur | 0,2 g |
| | |

| Phase B | |
|---|---|
| Polyglyceryl-3 Méthylglucose Distéarate (Tego care 450 | |
| de chez Goldschmidt) | 1,5 g |
| Stearate de glycéryle | 1,5 g |
| Alcool stéarylique | 1 g |
| Acide myristique | 2 g |
| Huile d'abricot | 7 g |
| Stéarate d'isocétyle | 8 g |
| Conservater qs | |
| Cyclohexasiloxane | 2 g |
| Diméthiconol | 1 g |
| | |

| Phase C | |
|---|---|
| Acide polyacrylique réticulé | 0,4 g |
| Eau | 15 g |
| | |

| Phase D | |
|---|---|
| Gomme de Xanthane | 0,2 g |
| Eau | 7 g |
| | |

| Phase E | |
|---|---|
| Triéthanolamine | 0,4 g |
| Eau | 1 g |
| | |

| Phase F | |
|---|---|
| Microparticules de l'exemple 4 | 8 g |

L'émulsion est préparée selon les techniques connues d'émulsification.
La composition appliquée sur la peau confère de bonnes propriétés d'hydratation et de douceur.

## Revendications

1. Microcapsules comprenant une enveloppe polymérique et/ou cireuse encapsulant un milieu aqueux contenant un polymère acrylique à groupement de type phosphorylcholine.

2. Microcapsules selon la revendication1, **caractérisée par le fait que** le polymère acrylique à groupement de type phosphorylcholine est un polymère ayant un squelette acrylique et comprenant des groupements pendants contenant un groupement de formule (I) suivante : 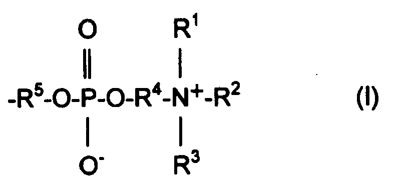 dans laquelle R¹, R² et R³ désignent indépendamment un groupe alkyle ayant de 1 à 8 atomes de carbone ; R⁴ désigne -(CH₂-CHR₆O)ₘ-(CH₂-CHR₆)ₚ- avec R₆ désignant un atome d'hydrogène, un groupe méthyl ou éthyl, et m désigne un nombre entier allant de 0 à 10 et p désigne un nombre entier allant de 1 à 2 ; R⁵ désigne -(CH2)_{g}-, g étant un nombre entier allant de 2 à 10.

3. Microscapsules selon l'une quelconque des revendications précédentes, **caractérisées par le fait que** le polymère acrylique à groupement de type phosphorylcholine est un polymère obtenu par polymérisation d'un monomère acrylique répondant à la formule (II) suivante : 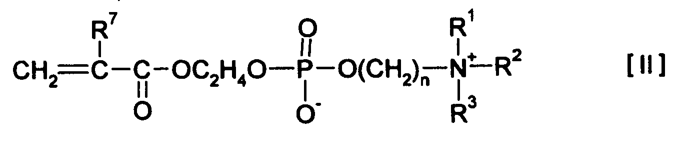 dans laquelle R¹, R² et R³ désignent indépendamment un groupe alkyle ayant de 1 à 8 atomes de carbone ; n représente un nombre entier allant de 2 à 4 ; R⁷ désigne un atome d'hydrogène ou un groupe méthyle.

4. Microcapsules selon la revendication précédente, **caractérisée par le fait que** le monomère acrylique de formule (II) est le 2-(méth)acryloyloxyéthyl-2'-(triméthylammonio)éthyl phosphate.

5. Microcapsules selon l'une quelconque des revendications 3 et 4, **caractérisées par le fait que** le polymère acrylique à groupement de type phosphorylcholine est un polymère obtenu par polymérisation d'un monomère acrylique de formule (II) et éventuellement d'un ou plusieurs monomères additionnels.

6. Microcapsules selon la revendication précédente, **caractérisées par le fait que** le monomère additionnel est choisi parmi le (méth)acrylate de méthyle, le (méth)acrylate d'éthyle, le (méth)acrylate de butyle, l'acide (méth)acrylique, le (méth)acrylamide, le (méth)acrylate de 2-hydroxyéthyle, l'éthyl vinyl éther, le butyl vinyl éther, la N-vinylpyrrolidone, le chlorure de vinyle, l'éthylène, l'isobutylène, l'acrylonitrile, le styrène, le méthyl styrène, le chlorométhyl styrène.

7. Microcapsules selon l'une quelconque des revendications 3 à 6, **caractérisées par le fait que** le polymère acrylique à groupement de type phosphorylcholine comprend de 40 à 100 % en mole d'unités issues du monomère acrylique de formule (II) et de 0 à 60 % en mole d'unités issues de monomère additionnel.

8. Microcapsules selon l'une quelconque des revendications précédentes, **caractérisées par le fait que** le polymère acrylique à groupement de type phosphorylcholine est choisi parmi l'homopolymère de 2-(méthacryloyloxyéthyl) phosphorylcholine, le copolymère 2-(méthacryloyloxyéthyl)phosphorylcholine/méthacrylate de butyle, le copolymère 2-(méthacryloyloxyéthyl)phosphorylcholine/chlorure de 2-hydroxy-3-méthacryloyloxypropyltriméthylammonium, le terpolymère de 2-(méthacryloyloxyéthyl) phosphorylcholine/méthacrylate de butyle/méthacrylate de sodium, le copolymère 2-(méthacryloyloxyéthyl) phosphorylcholine/méthacrylate de stéaryle.

9. Microcapsules selon l'une quelconque des revendications précédentes, **caractérisées par le fait que** le polymère acrylique à groupement de type phosphorylcholine est choisi parmi l'homopolymère de 2-(méthacryloyloxyéthyl) phosphorylcholine ou le copolymère 2-(méthacryloyloxyéthyl)phosphorylcholine/méthacrylate de butyle.

10. Microcapsules selon l'une quelconque des revendications précédentes, **caractérisées par le fait que** le polymère acrylique à groupement de type phosphorylcholine est l'homopolymère de 2-(méthacryloyloxyéthyl)phosphorylcholine.

11. Microcapsules selon l'une quelconque des revendications précédentes, **caractérisées par le fait que** le polymère acrylique à groupement de type phosphorylcholine a un poids moléculaire en poids allant de 50 000 à 1 000 000, et préférentiellement allant de 80 000 à 800 000.

12. Microcapsules selon l'une quelconque des revendications précédentes, **caractérisées par le fait que** le polymère acrylique à groupement de type phosphorylcholine est présent dans le milieu aqueux encapsulé dans les microcapsules en une teneur allant de 0,1 % à 40 % en poids, par rapport au poids total de la phase aqueuse encapsulée, de préférence allant de 1 % à 40 % en poids, de préférence encore allant de 2 % à 40 % en poids, de préférence allant de 5 % à 40 % en poids, préférentiellement allant de 5 % à 30 % en poids, et plus préférentiellement allant de 10 % à 25 % en poids.

13. Microcapsules selon l'une quelconque des revendications précédentes, **caractérisées par le fait que** l'enveloppe comprend un polymère insoluble dans l'eau.

14. Microcapsules selon l'une quelconque des revendications précédentes, **caractérisées par le fait que** l'enveloppe comprend un polymère insoluble dans l'eau choisi parmi :
- la polycaprolactone ;
- les poly (alkylène C2-C6 adipate) comme le poly(éthylène adipate) ou le poly(butylène adipate) ;
- les polyesters polyols d'acide adipique et de butanediol comme le polyester d'acide adipique, de 1,4-butanediol et de 2-éthyl-2-(hydroxyméthyl)-1,3-propanediol ;
- les esters de cellulose et d'au moins un acide carboxylique en C1-4, comme l'acétate de cellulose, l'acétopropionate de cellulose ;
- les poly(ortho ester) obtenus par polycendensation de polyol, de diol lactide et de 3,9-diéthylidène-2,4,8,10-tétraoxaspiro-[5.5]-undécane ;
- les polymères blocs poly(éthylèneglycol)-poly(butylene téréphatale) ;
- les copolymères polyéthylène glycol téréphtalate/polybutylène téréphatale ;
- les copolymères de styrène et d'anhydride maléique, les copolymères de styrène et d'acide acrylique, les terpolymères séquencés styrène-éthylène/butylène-styrène, les terpolymères séquencés styrène-éthylène/propylène-styrène, et les terpolymères d'éthylène, d'acétate de vinyle et d'anhydride maléique.

15. Microcapsules selon l'une quelconque des revendications précédentes, **caractérisées par le fait que** l'enveloppe comprend un polymère insoluble dans l'eau choisi parmi l'acétobutyrate de cellulose et l'acétopropionate de cellulose.

16. Microcapsules selon l'une quelconque des revendications précédentes, **caractérisées par le fait que** l'enveloppe comprend une cire.

17. Microcapsules selon l'une quelconque des revendications précédentes, **caractérisées par le fait que** l'enveloppe comprend une cire choisie parmi la cire d'abeille, la cire d'abeille polyglycérolée, les huiles végétales hydrogénées, la paraffine de point de fusion supérieur à 45 °C, et les cires de silicone.

18. Microcapsules selon l'une quelconque des revendications précédentes, **caractérisées par le fait que** le milieu aqueux encapsulé comprend un polymère hydrosoluble additionnel.

19. Microcapsules selon l'une quelconque des revendications précédentes, **caractérisées par le fait que** le polymère hydrosoluble additionnel est choisi parmi l'alcool polyvinylique), la polyvinylpyrrolidone, la carboxyméthylcellulose, les poly(acide carboxylique) et les dérivés réticulés de ceux-ci, et les gommes naturelles telles que les xanthanes, l'amidon, l'alginate de sodium, les pectines, le chitosane, le guar, le caroube, le carraghénane, l'acide hyaluronique.

20. Microcapsules selon l'une quelconque des revendications précédentes, **caractérisées par le fait que** le milieu aqueux encapsulé comprend un polyol miscible à l'eau à 25 °C choisi parmi le glycérol, l'éthylène glycol, le propylène glycol , le butylène glycol, le pentylène glycol.

21. Microcapsules selon l'une quelconque des revendications précédentes, **caractérisées par le fait que** le rapport en poids du milieu aqueux encapsulé à l'enveloppe de celles-ci est compris entre 0,1/1 et 50/1 et de préférence entre 0,5/1 et 10/1.

22. Microcapsules selon l'une quelconque des revendications précédentes, **caractérisées par le fait que** les microcapsules ont un diamètre moyen compris entre 1 µm et 1000 µm, et de préférence entre 1 et 80 µm.

23. Procédé de fabrication des microcapsules selon l'une quelconque des revendications précédentes, comportant les étapes successives suivantes :
(a) solubilisation d'au moins un polymère acrylique à groupement de type phosphorylcholine dans un milieu aqueux,
(b) émulsification de la solution aqueuse obtenue dans l'étape (a) dans une solution d'au moins un polymère insoluble dans l'eau et/ou d'au moins une cire dans un solvant organique non miscible à l'eau,
(c) émulsification de l'émulsion primaire eau-dans-huile obtenue dans l'étape (b) dans une solution aqueuse contenant de préférence un agent stabilisant de l'émulsion,
(d) élimination du solvant organique par évaporation donnant une suspension aqueuse de microcapsules.

24. Composition comprenant, dans un milieu physiologiquement acceptable, les microcapsules à coeur aqueux et à enveloppe polymérique et/ou cireuse selon l'une quelconque des revendications 1 à 22.

25. Composition selon la revendication précédente, **caractérisée par le fait qu'**elle comprend au moins un ingrédient choisi parmi l'eau, les solvants miscibles à l'eau à 25 °C, les huiles, les émulsionnants, les cires, les charges, les pigments, les matières colorantes, les séquestrants, les parfums, les épaississants, les conservateurs, les actifs cosmétiques ou dermatologiques, les filtres UV, les agents hydratants, les polymères filmogènes.

26. Procédé non thérapeutique de soin ou de maquillage des matières kératiniques comprenant l'application sur les matières kératiniques d'une composition selon l'une quelconque des revendications 24 et 25.

## Claims

1. Microcapsules comprising a polymeric and/or waxy envelope encapsulating an aqueous medium comprising an acrylic polymer comprising a group of phosphorylcholine type.

2. Microcapsules according to Claim 1, **characterized in that** the acrylic polymer comprising a group of phosphorylcholine type is a polymer having an acrylic backbone and comprising pendent groups comprising a group of following formula (I): 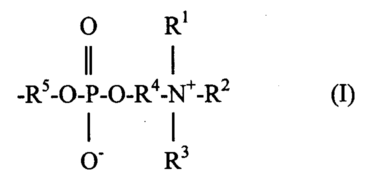 in which R¹, R² and R³ independently denote an alkyl group having from 1 to 8 carbon atoms; R⁴ denotes -(CH₂-CHR₆O)ₘ-(CH₂-CHR₆)ₚ- with R₆ denoting a hydrogen atom or a methyl or ethyl group and m denoting an integer ranging from 0 to 10 and p denoting an integer ranging from 1 to 2 ; R⁵ denotes -(CH₂)_{g}-, g being an integer ranging from 2 to 10.

3. Microcapsules according to either one of the preceding claims, **characterized in that** the acrylic polymer comprising a group of phosphorylcholine type is a polymer obtained by polymerization of an acrylic monomer corresponding to the following formula (II): 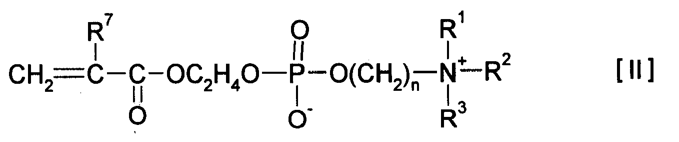 in which R¹, R² and R³ independently denote an alkyl group having from 1 to 8 carbon atoms; n represents an integer ranging from 2 to 4; R⁷ denotes a hydrogen atom or a methyl group.

4. Microcapsules according to the preceding claim, **characterized in that** the acrylic monomer of formula (II) is 2-(meth)acryloyloxyethyl 2'-(trimethylammonio)-ethyl phosphate.

5. Microcapsules according to either one of Claims 3 and 4, **characterized in that** the acrylic polymer comprising a group of phosphorylcholine type is a polymer obtained by polymerization of an acrylic monomer of formula (II) and optionally of one or more additional monomers.

6. Microcapsules according to the preceding claim, **characterized in that** the additional monomer is chosen from methyl (meth)acrylate, ethyl (meth)acrylate, butyl (meth)acrylate, (meth)acrylic acid, (meth)acrylamide, 2-hydroxyethyl (meth)acrylate, ethyl vinyl ether, butyl vinyl ether, N-vinylpyrrolidone, vinyl chloride, ethylene, isobutylene, acrylonitrile, styrene, methylstyrene or (chloromethyl)styrene.

7. Microcapsules according to any one of Claims 3 to 6, **characterized in that** the acrylic polymer comprising a group of phosphorylcholine type comprises from 40 to 100 mol% of units resulting from the acrylic monomer of formula (II) and from 0 to 60 mol% of units resulting from additional monomer.

8. Microcapsules according to any one of the preceding claims, **characterized in that** the acrylic polymer comprising a group of phosphorylcholine type is chosen from 2-(methacryloyloxyethyl)phosphorylcholine homopolymer, 2-(methacryloyloxyethyl)phosphorylcholine/butyl methacrylate copolymer, 2-(methacryloyloxyethyl)-phosphorylcholine/2-hydroxy-3-methacryloyloxypropyltrimethylammonium chloride copolymer, 2-(methacryloyloxyethyl)phosphorylcholine/butyl methacrylate/sodium methacrylate terpolymer or 2-(methacryloyloxyethyl)-phosphorylcholine/stearyl methacrylate copolymer.

9. Microcapsules according to any one of the preceding claims, **characterized in that** the acrylic polymer comprising a group of phosphorylcholine type is chosen from 2-(methacryloyloxyethyl)phosphorylcholine homopolymer or 2-(methacryloyloxyethyl)phosphorylcholine/butyl methacrylate copolymer.

10. Microcapsules according to any one of the preceding claims, **characterized in that** the acrylic polymer comprising a group of phosphorylcholine type is 2-(methacryloyloxyethyl)phosphorylcholine homopolymer.

11. Microcapsules according to any one of the preceding claims, **characterized in that** the acrylic polymer comprising a group of phosphorylcholine type has a weight-average molecular weight ranging from 50 000 to 1 000 000 and preferably ranging from 80 000 to 800 000.

12. Microcapsules according to any one of the preceding claims, **characterized in that** the acrylic polymer comprising a group of phosphorylcholine type is present in the aqueous medium encapsulated in the microcapsules in a content ranging from 0.1% to 40% by weight, with respect to the total weight of the encapsulated aqueous phase, preferably ranging from 1% to 40% by weight, more preferably ranging from 2% to 40% by weight, preferably ranging from 5% to 40% by weight, preferentially ranging from 5% to 30% by weight, and more preferentially ranging from 10% to 25% by weight.

13. Microcapsules according to any one of the preceding claims, **characterized in that** the envelope comprises a water-insoluble polymer.

14. Microcapsules according to any one of the preceding claims, **characterized in that** the envelope comprises a water-insoluble polymer chosen from:
- polycaprolactone;
- poly(C₂-C₆ alkylene adipate)s, such as poly(ethylene adipate) or poly(butylene adipate);
- polyester polyols of adipic acid and of butanediol, such as the polyester of adipic acid, of 1,4-butanediol and of 2-ethyl-2-(hydroxymethyl)-1,3-propanediol;
- esters of cellulose and of at least one C₁-C₄ carboxylic acid, such as cellulose acetate or cellulose acetate/propionate;
- poly(ortho ester)s obtained by polycondensation of polyol, of diol lactide and of 3,9-diethylidene-2,4,8,10-tetraoxaspiro[5.5]undecane;
- poly(ethylene glycol)-poly(butylene terephthalate) block polymers;
- poly(ethylene glycol terephthalate)/poly(butylene terephthalate) copolymers;
- copolymers of styrene and of maleic anhydride, copolymers of styrene and of acrylic acid, styrene-ethylene/butylene-styrene block terpolymers, styrene-ethylene/propylene-styrene block terpolymers and terpolymers of ethylene, of vinyl acetate and of maleic anhydride.

15. Microcapsules according to any one of the preceding claims, **characterized in that** the envelope comprises a water-insoluble polymer chosen from cellulose acetate/butyrate and cellulose acetate/propionate.

16. Microcapsules according to any one of the preceding claims, **characterized in that** the envelope comprises a wax.

17. Microcapsules according to any one of the preceding claims, **characterized in that** the envelope comprises a wax chosen from beeswax, polyglycerolated beeswax, hydrogenated vegetable oils, paraffin wax with a melting point of greater than 45°C and silicone waxes.

18. Microcapsules according to any one of the preceding claims, **characterized in that** the encapsulated aqueous medium comprises an additional water-soluble polymer.

19. Microcapsules according to any one of the preceding claims, **characterized in that** the additional water-soluble polymer is chosen from poly(vinyl alcohol), polyvinylpyrrolidone, carboxymethylcellulose, poly(carboxylic acid)s and the crosslinked derivatives of these, and natural gums, such as xanthans, starch, sodium alginate, pectins, chitosan, guar, locust bean, carrageenan or hyaluronic acid.

20. Microcapsules according to any one of the preceding claims, **characterized in that** the encapsulated aqueous medium comprises a polyol which is miscible with water at 25°C chosen from glycerol, ethylene glycol, propylene glycol, butylene glycol or pentylene glycol.

21. Microcapsules according to any one of the preceding claims, **characterized in that** the ratio by weight of the encapsulated aqueous medium to the envelope of the microcapsules is between 0.1/1 and 50/1 and preferably between 0.5/1 and 10/1.

22. Microcapsules according to any one of the preceding claims, **characterized in that** the microcapsules have a mean diameter of between 1 µm and 1000 µm and preferably between 1 and 80 µm.

23. Process for the manufacture of the microcapsules according to any one of the preceding claims, comprising the following successive stages:
(a) dissolution of at least one acrylic polymer comprising a group of phosphorylcholine type in an aqueous medium,
(b) emulsification of the aqueous solution obtained in stage (a) in a solution of at least one water-insoluble polymer and/or of at least one wax in a water-immiscible organic solvent,
(c) emulsification of the water-in-oil primary emulsion obtained in stage (b) in an aqueous solution preferably comprising an agent for stabilizing the emulsion,
(d) removal of the organic solvent by evaporation, giving an aqueous suspension of microcapsules.

24. Composition comprising, in a physiologically acceptable medium, the microcapsules comprising an aqueous core and comprising a polymeric and/or waxy envelope according to any one of Claims 1 to 22.

25. Composition according to the preceding claim, **characterized in that** it comprises at least one ingredient chosen from water, solvents which are miscible with water at 25°C, oils, emulsifying agents, waxes, fillers, pigments, coloring materials, sequestering agents, fragrances, thickeners, preservatives, cosmetic or dermatological active principles, UV screening agents, moisturizing agents or film-forming polymers.

26. Nontherapeutic method for caring for or making up keratinous substances comprising the application, to keratinous substances, of a composition according to either one of Claims 24 and 25.

## Patentansprüche

1. Mikrokapseln, die eine Polymerhülle und/oder Wachshülle aufweisen, die ein wässriges Medium einkapselt, das ein Acrylpolymer mit einer Gruppe vom Phosphorylcholin-Typ enthält.

2. Mikrokapseln nach Anspruch 1, **dadurch gekennzeichnet, dass** das Acrylpolymer mit einer Gruppe vom Phosphorylcholin-Typ ein Polymer ist, das ein Acrylskelett aufweist und Seitengruppen besitzt, die eine Gruppe der folgenden Formel (I) aufweisen: 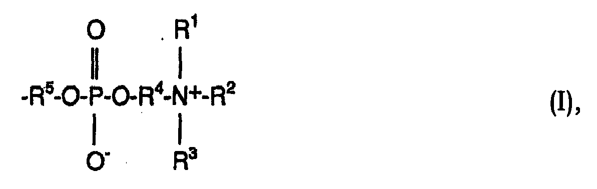 worin die Gruppen R¹, R² und R³ unabhängig voneinander eine Alkylgruppe mit 1 bis 8 Kohlenstoffatomen bedeuten; R⁴ die Gruppe -(CH₂-CHR₆O)ₘ-(CH₂-CHR₆)ₚ- ist, wobei R₆ ein Wasserstoffatom, eine Methylgruppe oder eine Ethylgruppe bedeutet und m 0 oder eine ganze Zahl von 1 bis 10 ist und p eine ganze Zahl von 1 bis 2 bedeutet; R⁵ -(CH2)g- ist, wobei g eine ganze Zahl von 2 bis 10 bedeutet.

3. Mikrokapseln nach einem der vorhergehenden Ansprüche, **dadurch gekennzeichnet, dass** das Acrylpolymer mit einer Gruppe vom Phosphorylcholin-Typ ein Polymer ist, das durch Polymerisation eines Acrylmonomers der folgenden Formel (II) erhalten wird: 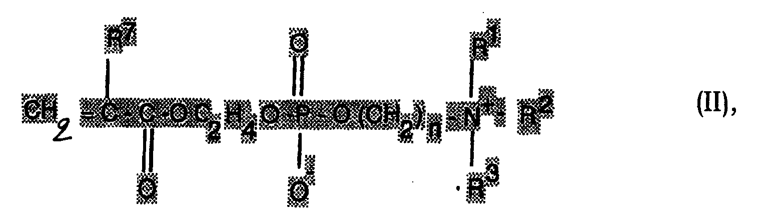 worin die Gruppen R¹, R² und R³ unabhängig voneinander eine Alkylgruppe mit 1 bis 8 Kohlenstoffatomen bedeuten; n eine ganze Zahl im Bereich von 2 bis 4 ist; und R⁷ ein Wasserstoffatom oder eine Methylgruppe bedeutet.

4. Mikrokapseln nach dem vorhergehenden Anspruch, **dadurch gekennzeichnet, dass** das Acrylmonomer der Formel (II) das 2-Methacryloyloxyethyl-2'-(trimethylammonio)ethyl-phosphat ist.

5. Mikrokapseln nach einem der Ansprüche 3 und 4, **dadurch gekennzeichnet, dass** das Acrylpolymer mit einer Gruppe vom Phosphorylcholin-Typ ein Polymer ist, das durch Polymerisation eines Acrylmonomers der Formel (II) und gegebenenfalls eines oder mehrerer ergänzender Monomere erhalten wird.

6. Mikrokapseln nach dem vorhergehenden Anspruch, **dadurch gekennzeichnet, dass** das ergänzende Monomer unter Methyl-(meth)acrylat, Ethyl(meth)acrylat, Butyl(meth)acrylat, (Meth)-acrylsäue, (Meth)acrylamid, 2-Hydroxyethyl(meth)acrylat, Ethylvinylether, Butylvinylether, N-Vinylpyrrolidon, Vinylchlorid, Ethylen, Isobutylen, Acrylnitril, Styrol, Methylstyrol, Chlormethylstyrol ausgewählt ist.

7. Mikrokapseln nach einem der Ansprüche 3 bis 6, **dadurch gekennzeichnet, dass** das Acrylpolymer mit einer Gruppe vom Phosphorylcholin-Typ 40 bis 100 Mol-% Einheiten, die von dem Acrylmonomer der Formel (II) stammen, und 0 bis 60 Mol-% Einheiten aufweist, die von dem ergänzenden Monomer abgeleitet sind.

8. Mikrokapseln nach einem der vorhergehenden Ansprüche, **dadurch gekennzeichnet, dass** das Acrylpolymer mit einer Gruppe vom Phosphorylcholin-Typ ausgewählt ist unter: dem Homopolymer von 2-(Methacryloyloxyethyl)-phosphorylcholin, dem 2-(Methacryloyloxyethyl)-phosphorylcholin/Butylmethacrylat-Copolymer, dem 2-(Methacryloyloxyethyl)-phosphorylcholin/2-Hydroxy-3-methacryloyloxypropyltrimethylammoniumchlorid-Copolymer, dem 2-(Methacryloyloxyethyl)-phosphorylcholin/Butylmethacrylat/Natriummethacrylat-Terpolymer, dem 2-(Methacryloyloxyethyl)-phosphorylcholin/Stearylmethacrylat-Copolymer.

9. Mikrokapseln nach einem der vorhergehenden Ansprüche, **dadurch gekennzeichnet, dass** das Acrylpolymer mit einer Gruppe vom Phosphorylcholin-Typ unter dem 2-(Methacryloyloxyethyl)-phosphorylcholin-Homopolymer oder dem 2-(Methacryloyloxyethyl)-phosphorylcholin/Butylmethacrylat-Copolymer ausgewählt ist.

10. Mikrokapseln nach einem der vorhergehenden Ansprüche, **dadurch gekennzeichnet, dass** das Acrylpolymer mit einer Gruppe vom Phosphorylcholin-Typ das 2-(Methacryloyloxyethyl)-phosphorylcholin-Homopolymer ist.

11. Mikrokapseln nach einem der vorhergehenden Ansprüche, **dadurch gekennzeichnet, dass** das Acrylpolymer mit einer Gruppe vom Phosphorylcholin-Typ eine gewichtsmittlere Molmasse von 50 000 bis 1 000 000 und vorzugsweise 80 000 bis 800 000 aufweist.

12. Mikrokapseln nach einem der vorhergehenden Ansprüche, **dadurch gekennzeichnet, dass** das Acrylpolymer mit einer Gruppe vom Phosphorylcholin-Typ in dem in den Mikrokapseln eingekapselten wässrigen Medium in einer Menge von 0,1 bis 40 Gew.-%, bezogen auf das Gesamtgewicht der eingekapselten wässrigen Phase, vorzugsweise 1 bis 40 Gew.-%, noch bevorzugter 2 bis 40 Gew.-%, vorzugsweise 5 bis 40 Gew.-%, bevorzugt 5 bis 30 Gew.-% und noch bevorzugter 10 bis 25 Gew.-% enthalten ist.

13. Mikrokapseln nach einem der vorhergehenden Ansprüche, **dadurch gekennzeichnet, dass** die Hülle ein in Wasser unlösliches Polymer enthält.

14. Mikrokapseln nach einem der vorhergehenden Ansprüche, **dadurch gekennzeichnet, dass** die Hülle ein in Wasser unlösliches Polymer enthält, das ausgewählt ist unter:
- Polycaprolacton;
- Poly(alkylen(C₂₋₆)adipaten), wie Poly(ethylenadipat) oder Poly(butylenadipat);
- Polyesterpolyolen von Adipinsäure und Butandiol, wie der Polyester von Adipinsäure, 1,4-Butandiol und 2-Ethyl-2-(hydroxymethyl)-1,3-propandiol;
- Estern von Cellulose und mindestens einer C₁₋₄-Carbonsäure, wie Celluloseacetat, Celluloseacetopropionat;
- Poly(orthoestern), die durch Polykondensation eines Polyols, eines Diollactids und von 3,9-Diethyliden-2,4,8,10-tetraoxaspiro-[5.5]-undecan erhalten werden;
- Blockpolymeren Poly(ethylenglycol)-poly(butylenterephthalat);
- Copolymeren Polyethylenglycolterephthalat/ Polybutylenterephthalat;
- Copolymeren von Styrol und Maleinsäureanhydrid, Copolymeren von Styrol und Acrylsäure, Sequenzterpolymeren Styrol-Ethylen/Butylen-Styrol, Sequenzterpolymeren Styrol-Ethylen/Propylen-Styrol und Terpolymeren von Ethylen, Vinylacetat und Maleinsäureanhydrid.

15. Mikrokapseln nach einem der vorhergehenden Ansprüche, **dadurch gekennzeichnet, dass** die Hülle ein in Wasser unlösliches Polymer enthält, das unter Celluloseacetobutyrat und Celluloseacetopropionat ausgewählt ist.

16. Mikrokapseln nach einem der vorhergehenden Ansprüche, **dadurch gekennzeichnet, dass** die Hülle ein Wachs enthält.

17. Mikrokapseln nach einem der vorhergehenden Ansprüche, **dadurch gekennzeichnet, dass** die Hülle ein Wachs enthält, das unter Bienenwachs, mehrfach mit Glycerin verethertem Bienenwachs, hydrierten pflanzlichen Ölen, Paraffin mit einem Schmelzpunkt über 45 °C und Siliconwachsen ausgewählt ist.

18. Mikrokapseln nach einem der vorhergehenden Ansprüche, **dadurch gekennzeichnet, dass** das eingekapselte wässrige Medium ein ergänzendes wasserlösliches Polymer enthält.

19. Mikrokapseln nach einem der vorhergehenden Ansprüche, **dadurch gekennzeichnet, dass** das ergänzende wasserlösliche Polymer unter Polyvinylalkohol, Polyvinylpyrrolidon, Carboxymethylcellulose, Poly(carbonsäure) und den vernetzten Derivaten dieser Verbindungen und natürlichen Gummen, wie Xanthanen, Stärke, Natriumalginat, Pektinen, Chitosan, Guar, Johannisbrotkernmehl, Carrageenan und Hyaluronsäure ausgewählt ist.

20. Mikrokapseln nach einem der vorhergehenden Ansprüche, **dadurch gekennzeichnet, dass** das eingekapselte wässrige Medium ein mit Wasser bei 25 °C mischbares Polyol enthält, das unter Glycerin, Ethylenglycol, Propylenglycol, Butylenglycol und Pentylenglycol ausgewählt ist.

21. Mikrokapseln nach einem der vorhergehenden Ansprüche, **dadurch gekennzeichnet, dass** das Gewichtsverhältnis des eingekapselten wässrigen Mediums und der Hülle der Mikrokapseln im Bereich von 0,1/1 bis 50/ 1 und vorzugsweise 0,5/ 1 bis 10/ 1 liegt.

22. Mikrokapseln nach einem der vorhergehenden Ansprüche, **dadurch gekennzeichnet, dass** die Mikrokapseln einen mittleren Durchmesser von 1 bis 1 000 µm und vorzugsweise 1 bis 80 µm aufweisen.

23. Verfahren zur Herstellung von Mikrokapseln nach einem der vorhergehenden Ansprüche, das die folgenden nacheinander ausgeführten Schritte umfasst:
(a) Solubilisieren mindestens eines Acrylpolymers mit einer Gruppe vom Phosphorylcholin-Typ in einem wässrigen Mediµm,
(b) Emulgieren der in Schritt (a) erhaltenen wässrigen Lösung in einer Lösung mindestens eines in Wasser unlöslichen Polymers und/oder mindestens eines Wachses in einem nicht mit Wasser mischbaren organischen Lösungsmittel,
(c) Emulgieren der in Schritt (b) erhaltenen primären Wasser-in-Öl-Emulsion in einer wässrigen Lösung, die vorzugsweise ein Stabilisierungsmittel für die Emulsion enthält,
(d) Entfernen des organischen Lösungsmittels durch Verdampfen, wodurch eine wässrige Suspension von Mikrokapseln gebildet wird.

24. Zusammensetzung, die in einem physiologisch akzeptablen Medium Mikrokapseln mit wässrigem Kern und einer Polymerhülle und/oder Wachshülle nach einem der Ansprüche 1 bis 22 enthält.

25. Zusammensetzung nach dem vorhergehenden Anspruch, **dadurch gekennzeichnet, dass** sie mindestens einen Bestandteil enthält, der unter Wasser, mit Wasser bei 25 °C mischbaren Lösungsmitteln, Ölen, Emulgatoren, Wachsen, Füllstoffen, Pigmenten, Farbmitteln, Maskierungsmitteln, Parfums, Verdickungsmitteln, Konservierungsmitteln, kosmetischen oder dermatologischen Wirkstoffen, UV-Filtern, Hydratisierungsmitteln und filmbildenden Polymeren ausgewählt ist.

26. Nicht-therapeutisches Verfahren für die Pflege oder zum Schminken von Keratinsubstanzen, das das Aufbringen einer Zusammensetzung nach einem der Ansprüche 24 und 25 auf die Keratinsubstanzen umfasst.
